# EUROPÄISCHE PATENTSCHRIFT

(11) **EP 1 555 983 B1**
(45) Veröffentlichungstag und Bekanntmachung des Hinweises auf die Patenterteilung: **06.10.2010**
(21) Anmeldenummer: 03772232.9
(22) Anmeldetag: 17.10.2003
(51) Int. Cl.: A61Q 19/00, A61K 8/04, A61K 8/89, A61K 8/46

(54) **Verschäumbare Zusammensetzungen**
Foamable compositions
Compositions moussantes

(30) Priorität: 31.10.2002 DE 10250755
(43) Veröffentlichungstag der Anmeldung: 27.07.2005
(73) Patentinhaber: Merz Pharma GmbH & Co. KGaA, 60318 Frankfurt (DE)
(72) Erfinder: PASPALEEVA-KÜHN, Valentina, 60323 Frankfurt am Main (BG); BEUTLER, Rolf, 64739 Höchst/Hummetroth (DE); HEBERER, Martina, 63073 Offenbach (DE)
(74) Vertreter: Müller, Claudia
(86) Internationale Anmeldenummer: PCT/EP2003/011528
(87) Internationale Veröffentlichungsnummer: WO 2004/039338

(56) Entgegenhaltungen:
- EP-A- 0 330 369
- WO-A-03/000207
- US-A- 5 169 623
- US-A- 6 071 975

## Beschreibung

Die vorliegende Erfindung betrifft eine, insbesondere verschäumbare, Zusammensetzung mit verbesserter Hautwirkung auf Basis einer O/W- Emulsion mit einer Kombination aus einem Emulgator auf Silikonbasis und einem insbesondere anionischen Tensid. Mit einer solchen Zusammensetzung können stabile Emulsionen mit oder ohne Treibgas bei der Anwendung verteilt oder wirksam verschäumt werden, welche dann leicht auf die Haut aufgetragen werden können und dort insbesondere eine besonders feine Verteilung der Zusammensetzung in Form einer Emulsion, z.B. als Schaum, erzielen und ein verbessertes Hautgefühl erzeugen. Die Erfindung betrifft ferner ein einfaches Herstellungsverfahren hierfür und die Anwendung zur Pflege, Behandlung oder milden Reinigung der Haut, insbesondere auch bei dysfunktionaler Haut.

### Stand der Technik

Verschäumbare Cremes wurden bisher auf verschiedene Weise hergestellt, wobei je nach Zusammensetzung Treibgase zwingend erforderlich sein können.

Auf Polysiloxan basierende Emulgatoren sind bekannt, vor allem zur Herstellung von Cremes, Lotionen etc. Sie verfügen allgemein über gute Emulgiereigenschaften und sind für versprühbare Produkte geeignet. Ein hier besonders bekanntes Produkt ist das unter der Bezeichnung Abil^{®} Care 85 bekannte Handelsprodukt, welches Bis- PEG/PPG-16/16 PEG/PPG-16/16- Dimethicone ist und als etwa 85%ige Lösung in einem Öl wie Capryl/Caprinsäure- Triglycerid erhältlich ist. Als solches wird es in Konzentrationen von 1,5 bis 3% eingesetzt. Bei der Herstellung von Emulsionen sollen allerdings aus Stabilitätsgründen nichtionische Co-Emulgatoren wie TEGO^{®} SMO 80 V (Polysorbat-80) (Kaltherstellung, z. B. für Sprays) oder Teginacid^{®} C (Ceteareth-25) (Heißherstellung z. B. für Cremes) sowie Stabilisatoren wie Xanthan Gum, TEGO^{®} Carbomer (Kaltherstellung für Emulsionen oder Sprays) bzw. Stearylalkohol, Glycerylstearat, Stearinsäure (Heißherstellung für Cremes) hinzugesetzt werden, vgl- SÖWF (2002), 128, Seite 22-31. Hier wird insbesondere auf die Schwierigkeiten bei der Herstellung von Sprühbarten Emulsionen hingewiesen (Seite 23), weshalb diese auch kalt und nur mit Hilfe spezieller Stabilisatoren (s. o.) angefertigt werden. Allerdings sind bis jetzt noch keine verschäumbaren Produkte hiermit beschrieben worden.

Die EP-B 1 014 916 betrifft alkalische Hautschutzcremes auf Seifenbasis, die zwingend mindestens eine C10-22-Fettsäure sowie Emulgatoren und Co-Emulgatoren enthält, die hergestellt werden durch Vermischen der auf 75 ° C erhitzten Fettphase nebst Fettsäure mit der auf 75 ° C erhitzten Wasserphase und anschließende Erhöhung des pH - Wertes auf ≥ 7,2.

In der EP- B 0 835 647 werden Zusammensetzungen in Form einer Gel- Emulsion beschrieben, welche neben einer Fettphase 0,1-2% eines gelbildenden Systems, enthaltend als Gel - Emulgator ein Homopolymer auf Acrylatbasis oder ein Copolymer auf Basis ethylenisch ungesättigter Monomere aufweisen. Dazu sollen noch Gelbildner wie Xanthan Gum, Carbomer^{®} etc. vorhanden sein. Nicht näher beschriebene Tenside dürfen nur bis zu maximal 2% enthalten sein. Das Produkt ist nachschäumend, d. h. es wird nicht als Schaum abgegeben.

Die DE 100 59 239 A1 betrifft Kosmetische und / oder pharmazeutische Emulsionen, enthaltend langkettige (Hydroxy)Fettsäuren wie Hydroxystearinsäuren sowie Öle und ggf. Emulgatoren, die aus verschiedenen Gruppen ausgewählt werden können. Unter anderen werden auch solche auf Polysiloxan - Polyalkyl - Basis allgemein genannt wobei gemäß den Beispielen keine erwähnt werden. Dort sind als nur Öle auf Siloxan - basis erwähnt, nämlich Di- / Cyclo- und *l* oder Cetyldimethicon. Sofern O/W oder insbesondere W/O - Emulsionen hergestellt werden, werden z. B. Polyglycerylderivate (Eumulgin VL 75 ®, Dehymuls PGPH ®) als Emulgator eingesetzt. Eine Verschäumbarkeit derartiger Substanzen ist nirgends angegeben.

DE 100 48 683 beschreibt W/O- Emulsionen, wobei als Emulgatoren Alkyl- (di)-Methiconcopolyole, z. B. Abil® EM 90, eingesetzt werden. Die so erhaltenden Mittel werden lediglich als versprühbar bezeichnet.

### Aufgabe der Erfindung

Aufgabe vorliegender Erfindung ist es daher, eine Zusammensetzung bereitzustellen, welche mit oder ohne Einsatz von Treibgasen leicht verteilbar, insbesondere verschäumbar, und leicht herstellbar ist , und auch ohne den speziellen Einsatz von komplexen Emulgator- bzw. Tensidgemischen oder zwingend Co- Emulgatoren / Stabilisatoren bzw. Gelbildnern zu stabilen Produkten führt.

### Lösung der Erfindung

Diese Aufgabe wird erfindungsgemäß gelöst durch eine Zusammensetzung, welche auf Basis einer ONV- Emulsion dadurch gekennzeichnet ist, dass sie 2 bis 50% einer Ölphase, 0,1 bis 10% eines oder mehrerer Emulgator(en)s auf Silikonbasis, 0,1 bis 20% eines oder mehrerer Tensid(e)s, ausgewählt aus anionischen Tensiden, 0 bis 40% Zusatzstoffe und als Rest Wasser aufweist. Die Zusammensetzung ist insbesondere verschäumbar.

### Erläuterung der Erfindung

Mit einer derartigen Zusammensetzung, die nicht auf alkalischer Seifengrundlage basiert, können überraschenderweise stabile Produkte auch ohne den Einsatz von speziellen Stabilisatoren bereitet werden, welche durch Verschäumbarkeit bei der Anwendung sehr gut verteilt, insbesondere verschäumbare sind. Dadurch wird aufgrund des im Vergleich zu herkömmlichen aufgetragenen Emulsionen sehr viel feiner verteilten Produktes eine verbesserte Wirkung auf der Haut erzielt und zusätzlich wegen des Silikon - Emulgators ein besonders reichhaltiges und glattes Hautgefühl erzeugt.

Das Mittel kann unterschiedliche Mengen an Wasser, Fettphase, Emulgator, und ggf. Zusatzstoffen enthalten. Hierbei werden dann je nach Anwendungszweck z. B. eine Lotion, ein Fluid oder eine Creme erhalten. Die Lotion kann z. B. 70% Wasser, die. Creme 50% Wasser aufweisen.

Es ist erfindungsgemäß daher nicht erforderlich, dass nichtionische Tenside, Fettsäuren inkorporiert werden. Diese sind daher bevorzugt, im wesentlichen, nicht enthalten.

Bevorzugt sind 0,1 bis 8%, insbesondere 0,5 bis 5% eines oder mehrerer Emulgatoren auf Silikonbasis; 0,1 bis 18%, insbesondere 0,3 bis 15% eines oder mehrere Tenside vorhanden.

Hierbei handelt es sich insbesondere um O/W- Emulgatoren auf Silikonbasis, in Mengen wie angegeben, wie nachfolgend beschrieben.

Die Menge an ölphase beträgt bevorzugt 3 bis 35%, insbesondere 3 bis 25% und ganz besonders bevorzugt 4 bis 25%.

Erfindungsgemäß sind als Emulgator ein oder mehrere Polysiloxan-Polyether- Copolymere (Dimethicon Copolyole) enthalten nämlich vor allem O/W- Emulgatoren. Hierunter sind ganz besonders ein oder mehrere Polyethylenglykol (PEG)-/ Polypropylenglykol(PPG)- Polysiloxan Copolymere mit unterschiedlichen Anteilen an PEG und PPG von jeweils 1-20 Einheiten bezogen auf einen Polyetherblock im Verhältnis 1:0 bis 0,9:1, insbesondere 1:0 bis 1:1 geeignet.

Hierzu gehören vor allem die PEG/PPG - Dimethicone in Form von O/W-Emulgatoren.

Dazu zählen insbesondere PEG/PPG-20/6 Dimethicone, Bis-PEG/PPG-20/20 Dimethicone, PEG-12 oder PEG-14 Dimethicone, PEG/PPG-14/4 oder 14/12 oder 14/14 oder 20/20 oder 18/18 oder 17/18 oder 15/15- Dimethicone, Bis PEG/PPG-16/16 PEG/PPG-16/16- Dimethicone oder Mischungen hiervon.

Besonders geeignet sind hier die unter der Marke Abil^{®} bekannten Produkte wie z.B. Abil^{®} Care 85 = Bis PEG/PPG-16/16 PEG/PPG-16/16- Dimethicone- (z. B. 85%ig in Caprylic/Capric Triglyceride).

Ferner sind auch die unter dem Namen Abil^{®} 8842 (1:0), 8843 (1:0), 8851 (1,4:4); 8852 (1,4:1,2); 8863 (1:1); sowie Dow Corning^{®} 193 (1:0) bekannten Silikon-Polyether besonders geeignet.

Diese silikonbasierenden Emulgatoren können als solche eingesetzt werden oder aber auch als Lösungen / Dispersionen in Ölen wie z. B. Triglyceriden, insbesondere mittelkettigen Triglyceriden oder auch geeigneten anderen Ölen wie Silikonölen. Hierin liegen sie z. B. in Mengen von 40 bis 90%, vorzugsweise 55 bis 90%, insbesondere sind 85%ige Lösungen in Caprytic/Capric Triglyceride geeignet.

Die Ölphase umfasst insbesondere übliche, vorzugsweise flüssige oder wachsartige Lipide. Diese können einzeln oder im Gemisch vorliegen. Insbesondere sind die folgenden Gruppen und Beispiele hierfür geeignet:
Kohlenwasserstoffe wie Squalen, Squalan, insbesondere auch flüssige Paraffine, Isoparaffine, Dioctylcyclohexane (Cetiol^{®} S), Isohexadecane (Arlamol^{®} HD);
Fettalkohole wie Oleylalkohol, Octyldodecanol (Eutanol^{®} G);
Fettsäureester, z.B. Isopropylfettsäureester (Palmitat, Myristat, Isostearat, Oleat), Decyl Oleate (Cetiol^{®} V), Hexyl Laurate, C 12 - 15 Alkyl Benzoate (Finsolv^{®} TN), Dicaprylyl Carbonate (Cetiol^{®} CC), Diester wie Dibutyladipate (Cetiol^{®} B), Propylenglykol Dipelargonate, verzweigte Fettsäureester wie PCL-liquid^{®} (Cetearyl Octanoate) oder Gemische wie Cetiol^{®} PGL (Hexyldecanol und Hexyldecyl Laurate) Fettalkoholether wie Dicaprylyl Ether (Cetiol^{®} OE) oder Fettakoholester wie C 12 - 13 Alkyl Lactate (Cosmacol^{®} ELI).

### Polyolfettsäureester wie Cetiol^{®} HE (PEG-7 Glyceryl Cocoate)

Triglyceride, insbesondere mittelkettige (Neutralöle) wie Caprylic/Ccapric Triglyceride (Miglyol^{®} 810, 812) sowie deren Polyolester wie Propylene Glycol Dicaprylate/Dicaprate (Miglyol^{®} 840)

Natürliche Fette und Öle wie Sonnenblumen-, Soja-, Pfirsichkern-, Aprikosenkern-, Traubenkem-, Ricinus-, Erdnuß-, Mandel-, Nerz-, Weizenkeim-, Avocadoöl, Shea Butter (z. B. Cetiol^{®} SB 45) oder Illipé Butter (Lipex^{®} 106).

### Natürliche flüssige Wachse, z.B. Jojobaöl oder dessen Substitut Oleyl Erucate (Cetiol^{®} J 600)

Silikonöle und -wachse, z.B. Polydimethylsiloxane wie Dow Corning Fluid^{®} 200 (Dimethicone), Cyclomethylsiloxane wie Dow Corning Fluid^{®} 345 (Cyclopentasiloxane), Phenylmethylpolysiloxane wie Phenyl Dimethicone (Abil^{®} AV 8853) oder Alkyl-Polymethylsiloxan-Copolymere wie Cetyl Dimethicone (Abil^{®} Wax 9801), Stearyl Dimethicone (Abil^{®} Wax 9800), Dialkoxydimethylpolysiloxane wie Stearoxy Dimethicone (Abil^{®} Wax 2434), Behenoxy Dimethicone (Abil^{®} Wax 2440) Wachse wie natürliches oder gebleichtes Bienenwachs (Lunacera^{®} alba), Kester^{®}Wachs K82H (C₂₀₋₄₀- Alkylstearat) oder Lunacera® M (Micro Wax) oder Kohlenwasserstoffwachse wie Lunacera^{®} P (Mineral Wax) sowie hydriertes Rizinusöl (Cutina^{®} HR) oder synthetische Wachse wie Cetylpalmitat (Cutina^{®} CP) oder Myristylmyristat (Crodamol^{®} MM), oder Stearylstearate (Crodamol^{®} SS). Die Fett(öl)phase ist bevorzugt ausgewählt aus Kohlenwasserstoffen, Fettalkoholen, -ethern und -estern, (Polyol)fettsäureestern, Triglyceriden, natürlichen Ölen, natürlichen Fetten, Wachsen ,Silikonölen, Silikonwachsen oder Mischungen hiervon. Insbesondere sind hier flüssige Paraffine, Triglyceride, Fettalkoholether, pflanzliche Öle, Silikonöl oder Mischungen hiervon bevorzugt.

Besonders bevorzugte Öl-Komponenten sind flüssige Paraffine, Fettsäureester wie Isopropylpalmitat oder -myristat, mittelkettige Triglyceride wie die vorgenannten Miglyole sowie die genannten natürlichen Fette und Öle, insbesondere Illipé Butter, Shea-Butter, Traubenkern-, Pfirsichkern-, Aprikosenkernöl, insbesondere Mischungen hiervon, sowie Jojobaöl, dessen Mischungen mit den vorgenannten Komponenten, wobei jeweils ca. 1-50% an Einzelkomponente, bezogen auf die Gesamtmenge vorhanden sind oder Fettalkoholether oder Fettalkohole oder Mischungen hiervon oder hydrierte Öle wie Perhydrosqualen einzeln oder kombiniert.

Insbesondere bevorzugt werden die genannten flüssigen Paraffine, Triglyceride, Fettalkoholether, Shea Butter und Silikonöle, auch in Kombination miteinander, wobei ca. 1 - 50% an Einzelkomponente bezogen auf die Gesamtmenge an Öl vorhanden sein können.

Ferner sind auch Silikonwachse besonders geeignet, vor allem auch Kombinationen hiervon und auch mit den vorgenannten Paraffinen, Fettalkoholether, und Shea Butter.

Ferner sind auch die o.g. Dioctylcyclohexane, Isohexadecane, insbesondere letztere, Silikonöle und -wachse besonders geeignet, vor allem auch Kombinationen hiervon und auch mit den vorgenannten Fettalkoholether und Trigyceriden. Diese können dann insbesondere auch mit den natürlichen Fetten, Ölen und Wachsen kombiniert werden.

Es ist ferner bevorzugt, wenn die Zusatzstoffe ausgewählt sind aus Wirkstoffen, Stabilisatoren, Farbstoffen, Parfümstoffen, Konsistenzreglern, Pflegestoffen, Co-Tensiden und Konservierungsmitteln. Insbesondere sind hiervon 0,01 bis 30%, vor allem 0,5 bis 20% enthalten.

Als Tenside sind anionische Tenside geeignet.

Hierfür sind die aus diesen Gruppen bekannten Tenside geeignet.

Bevorzugt werden als Tenside ein oder mehrere anionische Tenside gewählt. Hierzu gehören Alkylsulfate sowie Alkylethersulfate, insbesondere mit 1 - 6 Ethylenoxidgruppen im Molekül sowie Alkylsulfosuccinate, bzw. Alkylethoxysulfosuccinate, Alkylsulfosuccinamate, Alkylsarcosinate, Isethionate, Tauride, Ethercarbonsäuren, Alkylsulfonate sowie Alkylbenzotsulfonate, α-Olefinsulfonate, Alkylsulfoacetate, wobei im Alkylteil vor allem 8 - 22, bevorzugt bis 18 Kohlenstoffatome vorhanden sind.

Insbesondere sind hier als anionisches Tensid solche, ausgewählt aus Alkylsarcosinaten, Alkylsulfaten, Alkylethersulfaten, Alkylsulfosuccinaten, Alkylsulfosuccinnamaten, Isethionaten, bzw. deren Alkalisalze oder Mischungen hiervon geeignet. Diese weisen insbesondere 8 bis 22, bevorzugt 8 bis 16 Kohlenstoffe in der Alkylkette auf. Insbesondere sind hier Alkylethersulfate bevorzugt, die sich von Fettalkoholen mit 12 bis 18 Kohlenstoffatomen und einem Ethoxylierungsgrad von 2 bis 6 ableiten, wie z.B. Lauryl/Myristylethersulfat, Na-Salz (z.B. Texapon^{®} K 14 S spezial IS, Hansanol^{®} NS 243), Ammoniumlaurylethersulfat (Zetesol^{®} LA-2) oder Monoisopropanolammoniumlaurylethersulfat (Zetesol^{®} 2056) bzw. Alkylsulfate, z.B. Natriumlaurylsulfat (Texapon^{®} Z), Ammoniumlaurylsulfat (Texapon^{®} ALS, Sulfetal^{®} LA) oder Monoisopropanolammoniumlaurylsulfat (Sulfetal^{®} CJOT 60).

Ganz besonders bevorzugt sind ein oder mehrere Alkylsarcosinate wie Sodium Lauroyl, Cocoyl oder Oley) Sarcosinate, und ein oder mehrere Alkylsulfosuccinate wie unten beschrieben, wie oben genannt, oder Mischungen hiervon.

Vor allem sind Alkylsarcosinate wie Medialan® LD, Crodasinic^{®}LS 30, (Sodium Lauroyl Sarcosinate) oder Bernsteinsäurederivate, wie Alkylsulfosuccinate und Alkylsulfosuccinamate mit einem Alkylrest von 8 bis 22 Kohlenstoffatomen, z.B. Wallasol^{®}-L 29 (Disodium Laureth-3 Sulfosuccinate) auch alleine geeignet.

Vor allem sind die genannten Alkylsarcosinate bevorzugt.

Die o. g. Tenside können als solche oder in Verbindung mit geeigneten Lösemitteln eingesetzt werden, die sich nach der Beschaffenheit des Tensids richten. Es können somit Lösungen, insbesondere wässrige Lösungen von 20 - 90%, bevorzugt 25-70%, insbesondere 28 - 50% eingesetzt werden.

Ganz besonders bevorzugt beträgt das Verhältnis von Emulgator zu Tensid, jeweils bezogen auf die Gesamtmenge, 1:0,2 bis 1:15, vor allem aber 1:0,5 bis 1:10 und insbesondere 1:0,7 bis 1:7.

Die Menge an oberflächenaktiven Stoffen, insbesondere die Summe an Emulgator(en) + Tensid(e), kann auch geeigneter weise größer 1,4 %, oder auch größer 2% betragen.

Ganz besonders sind auch Zusammensetzungen geeignet, welche den oder die Emulgatoren in einer Gesamtmenge von 0,2 bis 5%, vor allem 0,5 bis 5%, insbesondere und das oder die Tenside in einer Gesamtmenge von 0,5 bis 15% enthalten.

Auch Zusammensetzungen mit 1 bis 30% Ölphase sind insbesondere geeignet.

Wie erwähnt ist es wünschenswert, wenn Zusatzstoffe, welche lipophilen und / oder hydrophilen Charakter aufweisen, enthalten sind. Hierzu gehören vor allem Wirkstoffe, z. B. 0,01 bis 80 %, insbesondere bis 50, vorzugsweise bis 25%, bezogen auf die Gesamtmenge an Zusatzstoffen oder 0,01 bis 10%, bezogen auf die Zusammensetzung Diese Wirkstoffe sind insbesondere ausgewählt aus Extrakten aus Pflanzen, diesen Extrakten entsprechenden synthetisch hergestellten Substanzen und analogen Derivaten hiervon, Vitaminen, sowie Haut beeinflussenden Wirkstoffen und Mischungen hiervon.

Extrakte aus Pflanzen sind beispielsweise solche aus Ylang Ylang, Kiefernnadel, Zypresse, Thymian, Minze, Limetten, Orangen, Grapefruit, Mandarine, Wacholder, Baldrian, Zitronenmelisse, Eukalyptus, Thymian, Palmarosa, Rosmarin, Lavendel, Rosenholz, Lemongras, Fichtennadel, Kiefernnadel, Ingwer-, Johannisbeer-, Lindenblüten-, Ringelblumen-, Magnolien-, Algen-, Aloe Vera-, Ananas-, Guave-, Echinacea-, Efeublätterxtrakt oder Mischungen hiervon.

Diese Extrakte können hergestellt werden z.B. durch Wasserdampfdestillafion. Hierdurch werden z.B. etherische Öle aus den genannten Pflanzen erhalten, welche besonders bevorzugt sind.

Die Extrakte können auch auf andere bekannte Weise durch z.B. Lösungsmittelextraktion (mit Alkoholen, Triglyceriden oder Kohlenwasserstoffen, Wasser, Gemischen hiervon) erhalten und als solche dann eingesetzt werden. Analoge synthetisch hergestellte Substanzen sind beispielsweise Terpene und Terpenoide wie Campher, Menthol, Cineol oder Mischungen hiervon.

Als Vitamine eignen sich z. B. Vitamin A, E, C bzw. geeignete Derivate hiervon wie Ester z.B. Palmitat, Acetat oder Phosphat.

Weiter geeignet sind Mineralstoffe und Spurenelemente wie Kupfer, Zink, bzw. deren Derivate wie Zincidone^{®} (Zink PCA), Zinkglukonat oder Kupfergluconat. Weiterhin können adstringierende und sebumregulierende Substanzen eingesetzt werden wie Acnacidol^{®} 101 (Propylene Glykol, Hydroxydecanoic Acid), Asebiol^{®} BT (Hydrolyzed Yeast Protein, Pyridoxine, Niacinamide, Glycerin, Panthenol, Propylene Glycol, Allatoin, Biotin), Lipacide^{®} C8C0 (Caproyl Collagen Aminoacids), Sebosoft^{®} (Glycerin, Aqua, PEG-8, Caprylyl Glycol, Sebacic Acid, Sodium Polyacrylate), Sepi Control A5 (Capryloylglycine, Methylglycine, Cinnamonum zeylanicum).

Ferner können als Zusatzwirkstoffe weitere Pflanzenextrakte enthalten sein, z. B. Birkenblätterextrakt, Aloe-Vera-Extrakt, Ringelblumen-, Hibiskus-, Klettenwurzel-Hamamelis-, Wassernabelkraut-, Algen-, Quitten-, Wasserlilien-, Zimtextrakt.

Es können auch kühlendelberuhigende Wirkstoffe wie Frescolat^{®} ML (Menthyl Lactate) oder Eashave^{®} (Sodium Hyaluronate, Wheat Germ Extrakt, Saccharomyces Cerevisiae Extrakt) inkorporiert werden.

Darüber hinaus sind als Zusatzstoffe durchblutungsfördernde Stoffe, z. B. Nikotinsäurederivate wie Methyl- oder Tocopherylnikotinat, Alpha- und Betahydroxysäuren und deren Derivate, z. B. Glykol-, Äpfel-, Zitronen-, Wein-, Milchsäure, Salicylsäure, Isopropylbenzylsalicylate, C12 - 13 Alkyl Lactate (Cosmacol^{®} ELI) oder auch antiphlogistische und antibakterielle Substanzen wie Triterpene, z. B. Ursolsäure, Glycyrrhizinsäure oder Glycyrrhetinsäure und deren Derivate, z. B. Stearyl Glycyrrhetinate, Kaliumglycyrrhinat; Pantothensäurederivate, z. B. D-Panthenol, Panthenyltriacetat; Allatoin; Bisabolol; Azulene, z. B. Cham-Azulene oder Guaj-Azulen; Phytosphingosine; Triclosan; Chlorhexidin-Derivate und/oder Antischuppenmittel, z. B. Climbazol oder Piroctone Olamine einsetzbar. Daneben können in den erfindungsgemäßen Zusammensetzungen auch antioxidativ sowie zellschützend wirkende Stoffe wie Polyphenole, Flavonoide, z. B. Rutin, Ferulasäure und deren Ester oder Isoflavone wie Soja-Isoflavone oder Coenzym Q 10 als wirksame Zusatzstoffe eingesetzt werden.

Weitere Wirkstoffe sind UV-Filter wie UVB, UVA und Breitbandfilter der folgenden Art:
UV-B Filter: Zimtsäureester, z.B. Ethylhexyl Methoxycinnamate (Eusolex^{®} 2292, Neo Heliopan^{®} AV, Parsol^{®} MCX), Isoamyl p-Methoxycinnamate (Neo Heliopan^{®} Galanga) sowie 4-Methylbenzyliden Camphor (Eusolex^{®} 6300), Paraaminobenzoesäure und -ester wie N, N-Dimethyl-4-aminobenzoesäure-2-ethylhexylester (Eusolex^{®} 6007, Octyl Dimethyl PABA), Homomenthylsacilylat (Homosalate, Eusolex^{®} HMS), Octyl Salicylate (Neo Heliopan^{®} OS), Octocrylene (Neo Heliopan^{®} 303), Phenylbenzimidazolesulfonic Acid (Neo Heolipan^{®} Hydro, Eusolex^{®} 232) oder Benzylidene Malonate Polysiloxane (Parsol^{®} SLX); UVA + UVB Filter für Breitbandabsorption wie Benzophenone-3 (Neo^{®} Heliopan BB, Eusolex^{®} 4360).
UV-A Filter wie Methyl Anthranilate (Neo Heliopan^{®} MA), Butyl Methoxydibenzoylmethane (Parsol^{®} 1789, Eusolex^{®} 9020), Methylene Bis-Benzotriazolyl Tetramethylbutylphenol (Tinosorb^{®} M), Bis-Ethylhexyloxyphenol Methoxyphenyltriazin (Tinosorb^{®} S), Disodium Phenyl Dibenzimidazole Tetrasulfonate (Neo Heliopan^{®} AP), 2-(4-Diethylamino-2-hydroxybenzoyl)benzoic Acid Hexylester (Uvinui^{®} A Plus)
Insbesondere bevorzugt sind Benzylidene Malonate Polysiloxane, Octyl- oder Isoamyl p- Methoxycinnamate, Octocrylene, 4-Methylbenzyliden Camphor, Homosalate und/oder Butyl Methoxydibenzoylmethane und/oder Benzophenone-3. Weiterhin geeignet sind anorganische UV-Filter wie Zinkoxid und Titandioxid insbesondere mikronisiert und/oder beschichtet, z.B. Z-Cote^{®}, Tioveil^{®}, Solaveil^{®} oder Unititamer^{®}T 40.

Dabei kann je nach beabsichtigtem Wirkungseffekt, wie z.B. Beruhigung, Verbesserung der Hautstruktur, Erhöhung der Durchblutung, Entspannung, Aromatherapie u.ä. eine geeignete Kombination an Wirkstoffen zugesetzt werden. Besonders bevorzugte Wirkstoffe sind etherische Öle aus Ylang Ylang, Lemongras, Minze, Orangen, Grapefruit, Limette, Mandarine, Zypresse, Thymian, Lavendel, Rosmarin, Ingwer, oder Mischungen hiervon.

Ferner werden vor allem auch die genannten Vitamine und/oder Panthenol eingesetzt, insbesondere in Kombination mit den Pflanzen-Extrakten oder etherischen Ölen.

Besonders erwünschte Wirkstoffe sind ausgewählt aus Vitaminen, UV-Filtern oder dermatologischen Wirkstoffen, z. B. Antimykolika wie Naftifin HCl, Terbinafin HCl oder antioxidativen, antibakteriellen und/oder sebumregulierenden Stoffen.

Die erfindungsgemäße Zusammensetzung kann darüber hinaus als weitere Zusatzstoffe Farbstoffe, Konditioniermittel, Pflegestoffe, Konservierungsstoffe, Parfümstoffe, Stabilisatoren, Konsistenzregler enthalten. Diese können insbesondere in folgenden Mengen vorliegen: Konditioniermittel, z.B. 0,01 bis 5% ; Stabilisator(en), z.B. 0,01 bis 5%, insbesondere 0,05 bis 3%, Konservierungsmittel, z.B.0,01 bis 5%, insbesondere 0,1 bis 5%; Konsistenzregler, z.B.0,01 bis 10%; Pflegestoffe, z.B. 0,01 bis 10%; Farbstoffe 0,0005 bis 3%, Parfümstoffe, z.B. 0,01 bis 5%.

Als Pflegestoffe (z.B. bevorzugt 0,01 bis 20%, insbesondere 0,01 bis 10%) können ein oder mehrere Lecithine oder Derivate hiervon wie insbesondere Lecithin, Phospholipide, wie Phosphatidylcholin, und Ceramide, wie z.B. Ceramid-2, -3, -6 sowie Sphingolipide, wie z.B. Proceramide^{®} L, Ceraderm^{®} S, Ceraveg^{®}.

Besonders geeignete Pflegestoffe sind auch Phytosterole (im wesentlichen Gemische aus β-Sitosterol, Campesterol und Stigmasterol) wie solche aus Rapsöl (Generol^{®} R).

Besonders bevorzugt sind Lecithin (z.B. Sojalecithin), Phosphatidylcholin, Phosphatidylserin oder- diethanolamin, die oben genannten Generole® oder Mischungen hiervon.

Weiterhin können Silikonöle z.B. Dimethicone oder Silikon -(Co)Polymere, z.B. Dow Corning^{®} HMW 2220 (Divinyldimethicone/Dimethicone Copolymer, C12-13 Pareth-3/C12-13 Pareth-23) Verwendung finden.

Weitere Pflegestoffe sind Feuchthaltemittel wie Glycerin, Propylenglykol oder Polyethylenglykole, Propylenglykol, Butylenglykol, Sorbitol oder Polymere, z.B. Polyquaternium-Typen wie Polyquaternium -39 (Merquat^{®} plus 3330), Proteine wie Collagen oder dessen Hydrolysate, Aminosäuren, Harnstoff, pflanzliche Proteine wie z.B. aus Weizen, Soja oder Mandel oder deren Hydrolysate, z.B. Tritisol^{®} (Hydrolyzed Wheat Protein), Promois^{®} WK (Hydrolyzed Keratin), Promois^{®} Milk (Hydrolyzed Casein), Wheat-Tein^{®} AA (Hydrotyzed Wheat Protein), Lactolan^{®} LS-5879 (Hydrolyzed milk protein), Polysaccharide wie z.B. Fucogel^{®} 1000 (Biosaccharide Gum-1), Glucosaminoglykane, z.B. Hyaluronsäure oder sulfatierte Glucosaminoglykane wie Chondroitinsulfat, Dermatansulfat, Keratansulfat, Heparansulfat, insbesondere deren Na-Salze oder Heparin-Na, Glukane, z.B. β-Glukan, z.B. Hafer β-Glukan (Drago-β-Glucan^{®}), Mannane wie Konjac Mannane, Algenextrakte, z.B. Seanamin^{®} SU oder handelsübliche Feuchhaltemittel wie z.B. Hydractin^{®} (Glycerin, Aqua, Disodium Adenosine Triphosphate, Algin, Carica Papaya) oder Aquaderm^{®} (Sodium PCA, Sodium Lactate, Fructose, Glycine, Niacinamid, Urea, Inositol), Salze, z.B. Natriumlactat, DL-2-Pyrrolidon-5-Carbonsäure, Na-Salz.

Bevorzugt sind Polyethylen-/ Propylenglykol oder Glycerin in Mengen von z.B.0,5-10%, insbesondere 2- 5%, sowie Polysaccharid- Verbindungen wie Fucogel^{®} 1000, Algenextrakte und/oder Hyaluronsäure, Na-Salz.

Als Stabilisatoren (z.B. bevorzugt 0,01 bis 5%) können z.B. Komplexbildner wie Trilon^{®}BD (EDTA, Na-Salz), Mittel zur pH- Einstellung, z.B. Zitronensäure, Natronlauge Lösungsmittel wie Propylenglykol oder Alkohole, Stärke, bzw. Stärke-Derivate oder Mischungen hiervon enthalten sein.

Die erfindungsgemäßen Zusammensetzungen sind insbesondere neutral und hautfreundlich und weisen insofern bevorzugt eine pH-Wert von < 7,2, insbesondere ≤ 7, auf, in Korrelation mit ggf. pH- empfindlichen Zusatz- bzw. Wirkstoffen.

Als Stärke, bzw. Stärke-Derivate sind insbesondere folgende Stoffe geeignet:
Reis-, Weizen-, Mais- und Kartoffelstärke.
Besonders bevorzugt sind hydrophobisch modifizierte Stärken wie Dry Flo^{®} AF (Corn Starch Modified), Aluminium Starch Octenylsuccinate (Dry Flo^{®} PC, Fluidamid^{®} DF 12), Hydroxypropyl Starch Phosphate Ester (Sturcture^{®} XL) oder dessen Gemische wie Natrasorb® HFB (Aluminium Starch Octenylsuccinate, Acrylates Copolymer, Magnesium Carbonate), ASO/MM3^{®} (Aluminium Starch Octenylsuccinate, Magnesium Myristate), Dry Flo^{®} Elite LL (Aluminium Starch Octenylsuccinate, Lauroyl Lysine), Facemat^{®} (Aluminium Starch Octenylsuccinate, Mica, Zea Mays (Corn) Starch, Silica, Titanium Dioxide, Zink Oxide).
Ganz besonders bevorzugt sind Dry Flo^{®} PC oder AF und Natrasorb^{®} HFB.

Weitere Stabilisatoren sind insbesondere anionische Co- Tenside wie Glutamate, z.B. Sodium Cocoyl Glutamate (Hostapon^{®} CCG) oder Mono- und Dialkylphosphate oder selbstemulgierendes Glycerylstearat.

Daneben können gegebenenfalls ein oder mehrere Konservierungsmittel und Konsistenzregler (bevorzugt z.B. je 0,01 bis 5 %) vorhanden sein. Geeignete Konservierungsmittel sind beispielsweise lodopropynylbutylcarbamat, DMDM Hydantoin, Phenoxyethanol und weitere gebräuchliche Konservierungsstoffe, wie z.B. Sorbin- und Dehydracetsäure und deren Salze, Methyldibromoglutanonitril, etc. oder Kombinationen hiervon, oder andere Säuren wie Benzoe- oder Salicylsäure, oder Benzylalkohol oder Ester wie p-Hydroxy-benzoesäureester, z.B. Methyl-, Ethyl-, Propyl-, Butyl-, Iso-Butylparaben, bevorzugt Methyl-oder Propylparaben oder Mischungen hiervon oder Climbazol oder geeignete Kombinationen der genannten Stoffe wie z.B. Methyl-, Propylparaben und Sorbinsäure in Frage. Insbesondere geeignete sind Mischungen, z.B. aus DMDM Hydantoin und lodopropynylbutylcarbamat wie Glydant^{®} plus oder aus Phenoxyethanol mit p-Hydroxybenzoesäureester wie Phenonip^{®}, Isothiazolinone, Methyldibromoglutanonitril, etc.

Als Konsistenzregler (z.B. bevorzugt 0,01 bis 5%) eignen sich Gelbildner wie Acrylamide oder Acrylate sowie Polysaccharide wie Keltrol^{®} (Xanthan Gum), Cellulose-Derivate wie Hydroxypropyl Methyl Cellulose (Methocel^{®} J12-MS) oder Ethylcellulose oder Silikate wie Magnesium Aluminium Silikat (Veegum^{®} HV). Bevorzugte Acrylamide sind Polyacrylamid, z.B. Flocare^{®} T 920 GC oder Polyacrylamidhaltge Gemische wie Sepigel^{®} 305 (Polyacrylamide, C13-14 Isoparaffin, Laureth-7), Sepigel^{®} 501 (Acrylamides Copolymer, Mineral Oil, C13-14 Isoparaffin, Polysorbate 85), Sepigel® 502 (C13-14 Isoparaffin, Isostearyl Isostearate, Sodium Polyacrylate, Polyacrylamide, Polysorbat 20), Creagel^{®} EZ DC (Polyacrylamide, Polydecene, Dimethioone Copolyol), Creagel^{®} EZ 5 (Polyacrylamide, Polydecene, Laureth-5).

Als Acrylate werden Carboxy-Vinyl-Mischpolymerisate mit hohem Molekulargewicht (1 - 3 Mio.) sowie deren Copolymere eingesetzt, insbesondere nach Neutralisierung durch Alkali. Bevorzugt sind die unter dem INCI-Namen Carbomer bekannten Carbopol^{®}-Typen, z.B. Carbopol^{®} 910, 934, 940, 941, 954, 980, 981, 2984, 5984 oder Carbopol^{®} ETD 2001, 2050 oder Synthalen^{®} K, L, M oder die bereits neutralisierten Carbomere wie PNC^{®} 400, 410, 430 (INCI: Sodium Carbomer). Es können auch Acrylat-Copolymere eingesetzt werden, z.B. Arylates/C10-30 Alkyl Acrylate Crosspolymer, bekannt als Carbopol^{®} 1342, 1382, ETD 2020, Pemulen^{®} TR-1, TR-2. Besonders bevorzugt sind Acrylamidalkylsulfonsäurederivate wie Ammonium Acryloyldimethyltaurate/VP Copolymer (Aristoflex^{®} AVC).

Weitere geeignete Konsistenzgeber sind z. B. Fettalkohole, wie Stearyl Alcohol (Lanette^{®} 18), Cetyl Alcohol (Lanette^{®} 16), Myristyl Alcohol (Lanette^{®} 14) oder Cetearyl Alcohol (Lanette^{®} O) ausgewählt werden. Ferner geeignet sind Glycerylester wie Glycerylstearat, insbesondere Glycerinmonostearat oder Glycerindistearat oder Gemische hiervon, z.B. Tegin^{®} M.

Weiterhin können als Konsistenzgeber auch die o. g. Wachse eingesetzt werden.

Besonders bevorzugte Konsistenzgeber sind die o.g. Fettalkohole wie Stearyl Alcohol (Lanette^{®} 18), Cetyl Alcohol (Lanett^{®} 16), Myristyl Alcohol (Lanette^{®} 14) oder Cetearyl Alcohol (Lanette^{®} O), insbesondere in Kombination mit Acrylaten wie Aristoflex^{®} AVC oder Carbomeren.

Geeignete Parfümstoffe (z.B. bevorzugt 0,01 bis 5%) sind z.B. die unter Wirkstoffen genannten etherischen Öle oder handelsübliche Parfümmischungen.

Als Farbstoffe (z.B. bevorzugt 0,01 bis 2%) werden vorzugsweise die für gattungsgemäße Produkte bekannten Komponenten gewählt wie z.B. Patentblau, Amidoblau, Orange RGL, Cochenillerot, Chinolingelb.

Die Art und Menge der Zusatzstoffe richtet sich nach der gewünschten Anwendungsform und dem gewünschten Zweck. Es ist bevorzugt, wenn höchstens 25%, bevorzugt 0,01 bis 20%, vor allem auch 0,1 bis 20% oder 1 bis 18 %, insbesondere 5 bis 15% an Zusatzstoffen vorhanden sind. Besonders bevorzugt werden Stabilisatoren, Farbstoffe, Antioxidantien, Parfümstoffe, Konsistenzregler, Pflegestoffe, ggf. Konservierungsmittel oder Mischungen hiervon gewählt, wobei Pflegestoffe und Stabilisatoren besonders bevorzugt werden. Bevorzugt werden Konservierungsstoffe, ggf. in Verbindung mit Wirkstoffen und/oder pH- Regulatoren als Zusatzstoffen.

Besonders bevorzugt sind 0,01 bis 25% Zusatzstoffe, welche vor allem ausgewählt sind aus Wirkstoffen, Pflegestoffen, Stabilisatoren, Farbstoffen, Parfümstoffen, Konsistenzreglern.

Hierunter sind ganz besonders Konsistenzregler wie Fettalkohole, Acrylatpolymere wie Aristoflex^{®} AVC oder Carbomere und/oder Acrylat Copolymere, Cellulosederivate, Stabilisatoren wie Stärke- Derivate, insbesondere Dry Flo^{®} AF Co- Tenside wie Glutamate, und/oder Feuchthaltemittel, Konditioniermittel geeignet. Insbesondere sind als Konsistenzregler Fettalkohole, Cellulosederivate oder Mischungen hiervon geeignet.

Als Pflegestoffe werden insbesondere Feuchthaltemittel wie Glycerin und/oder Polymere und/oder Polysaccharide eingesetzt.

Als Parfüm- bzw. Farbstoffe kommen vor allem Kompositionen, bzw. für Kosmetika gebräuchliche Farbstoffe infrage, als Wirkstoffe sind Vitamine sowie Pflanzenextrakte, UV-Filter, antioxidative Stoffe, Panthenol und/oder Allantoin und/oder Mineralstoffe und deren Derivate besonders bevorzugt.

Die erfindungsgemäße Zusammensetzung kann in für Emulsionen üblichen Vorrichtungen enthalten und hieraus abgegeben werden. Bei Austritt und Verreiben an/ mit Luft entsteht so das wie beschrieben fein verteilte, feinblasige bis ggf. schäumförmige Produkt auf der Haut.

Erfindungsgemäß kann die insbesondere verschäumbare Zusammensetzung bevorzugt als Schaum vorliegen. Hierzu kann diese z. B. in einem Schaumspender mit einem geeigneten Pumpenmechanismus oder auch zusammen mit einem geeigneten Treibgas in einer Aerosolvorrichtung enthalten sein. Hierbei wird das Produkt direkt als Schaum erhalten und ist nicht wie im oben genannten Stand der Technik beschrieben, nachschäumend. Die Aerosolvorrichtung kann einen Druckgasbehälter mit Treibmittel, eine Verteilervorrichtung mit Ventil, Sprühkopf sowie die Zusammensetzung aufweisen.

Als Treibgas eignen sich hierfür handelsübliche Gase oder Gasmischungen wie CO₂, N₂, N₂O oder Gemische hiervon wie CO₂:N₂O 1:1 bis 0,5:1. Ganz besonders bevorzugt werden Gase wie Butan/ Propan/ Isobutan oder Mischungen hiervon wie sie handelsüblich für Aerosole sind, z.B. eine 3-5:20-25:65-73 wie z.B. als Drivosol^{®} (35A) erhältlich. Das Gas kann dabei komprimiert in Mengen von 2 bis 20%, bevorzugt 3 - 13%, insbesondere 4 - 8%, bezogen auf das Fertigprodukt, vorliegen. Diese Mengenverhältnisse können jedoch je nach Gesamtmenge des Produktes bzw. Größe des Behälters auch in anderen Verhältnissen variiert werden. Geeignete Vorrichtungen zur Verabreichung der erfindungsgemäßen Zusammensetzung als Aerosol sind bekannt, ebenso solche zur treibgaslosen Applikation. Hierbei können beispielsweise mechanische, nach dem Prinzip der Luftpumpe arbeitende Schaumspender wie Squeeze Foamer (F2 Finger Pump Foamer, im Handel erhältlich), Rieke Dispensing von Fa. Englass oder Foamer der Fa. Airspray verwendet werden, wobei die fertige Emulsion jeweils in geeigneter Weise eingefüllt wird. Bevorzugt befindet sich die Zusammensetzung in herkömmlichen Spendergefäßen. Insbesondere wird sie aus einem Schaumspender oder einem mit Treibgas befüllten Aerosolbehältnis abgegeben.

### Herstellung

Die erfindungsgemäßen Zusammensetzungen werden hergestellt, indem man die Ölphase, den oder die Emulgatoren, ggf. zusammen mit temperaturstabilen öllöslichen Zusatzstoffen mischt und ggf. auf 50 bis 85° C erhitzt und sodann die wässrige, ggf. auf 50 bis 85° C erwärmte Phase, enthaltend Wasser, das oder die Tenside und ggf. temperaturstabile Zusatzstoffe, unter Rühren hinzufügt, und anschließend die entstandene Emulsion ggf. abkühlt und, sofern vorhanden, temperaturlabile Zusatzstoffe hinzufügt. Die so hergestellte Emulsion kann dann in bekannter Weise in geeignete Behältnisse zur Verabreichung z. B. aus üblichen Spendern, Abgabevorrichtungen oder -gefäßen, oder zur Abgabe in Schaumform in die oben beschriebenen Pumpenspender oder Aerosolbehälter gefüllt werden.

### Anwendung

Insbesondere kann die erfindungsgemäße Zusammensetzung angewendet werden zur Pflege, zur kosmetischen oder dermatologischen Behandlung von Haut zur Erfrischung, Belebung, Feuchtigkeitsregulation, Beruhigung oder zur milden Reinigung, vor allem auch bei dysfunktionaler, insbesondere trockener oder feuchtigkeitsarmer Haut, oder auch zum Schutz der Haut. Die Wirkung, welche durch entsprechende wie oben beschriebenen Zusatzstoffe unterstützt werden kann, wird insbesondere durch die besonders feine Verteilung der Zusammensetzung in Form einer Emulsion, z. B. insbesondere als Schaum erzielt, wobei darüber hinaus noch in unerwarteter Weise ein verbessertes Hautgefühl erzeugt wird durch den Einsatz des Emulgators zusammen mit den genannten Tensiden.

Wie erwähnt kann die Abgabe des Produktes mittels herkömmlicher Abgabegefäße, Spender, mittels Vorrichtung mit Pumpenmechanismus oder aus Aerosol- haltigen Behältern erfolgen. Es wird angenommen, ohne dass eine Festlegung auf eine derartige Erläuterung erfolgt, dass aufgrund der erfindungsgemäßen Kombination die physikalisch - chemischen Eigenschaften der Zusammensetzung derart ist, dass die Feinverteilung des Produktes bei der Anwendung durch Verreiben an / mit Luft / Aerosol, d. h. durch die Möglichkeit der Bildung von Schaum, erfolgt. Das Produkt kann daher als solches oder als Schaum appliziert werden.

### Beispiele

Die Erfindung wird anhand der nachfolgenden Beispiele näher erläutert.

Dabei bedeuten die einzelnen Inhaltsstoffe:
Trilon^{®} BD = Disodium EDTA
Abil^{®} Care 85 = Bis-PEG/PPG -16/16 PEGIPPG 16/16 Dimethicone, ca. 85%ig in Caprilyc/Capric Triglyceride
Oxynex^{®} 2004 = Propylene Glycol, BHT, Ascorbyl Palmitate, Glyceryl Stearate, Citric Acid
Höstapon^{®} CCG = Sodium Cocoyl Glutamate, ca. 30%ige wäßrige Lösung
Medialan^{®} LD = Sodium Lauroyl Sarcosinate, ca. 30%ige wäßrige Lösung
Phenonip^{®} = Phenoxyethanol, Methylparaben, Ethylparaben, Propylparaben, Butylparaben, Isobutylparaben
Aristoflex^{®} AVC = Ammonium Acryloyldimethyltaurate/VP Copolymer
Seanamin^{®} SU = Aqua, Sorbitol, Algae, Chondrus Crispus, Fucus vesiculosus, Algin;
Fucogel^{®} 1000 = Biosaccharide Gum-1
Merquat^{®} plus 3330 = Polyquaternium-39, ca. 9,5%ige wäßrige Lösung Dry Flo^{®} AF = Corn Starch Modified
PNC^{®} 430 = Sodium Carbomer
Hansahol^{®} NS 243 = Sodium Laureth Sulfate, ca. 28%ige wäßrige Lösung
Brij^{®} 721 = Steareth-21; Tween^{®} 80 = Polysorbate-80
Hostaphat^{®} KW 340 D = Triceteareth-4 Phosphate

Die Beispiele 1 bis 7 repräsentieren erfindungsgemäße Zusammensetzungen, welche alle eine gute Schaumbildung mit kleinporigem und nicht schnell zusammenfallendem Schaum und Stabilität zeigen.

Die Beispiele 8 bis 11 sind Vergleichsbeispiele wie nachfolgend beschrieben, enthaltend nicht ionische Tenside.

### Beispiel 1

| **Rohstoffe** | **100% Rezeptur** |
|---|---|
| Aqua purificata | 59,96 |
| Trilon^{®} BD | 0,05 |
| Glycerin | 7,00 |
| Zitronensäure | 0,04 |
| Abil^{®} Care 85 | 3,00 |
| Cetearyl Akohol | 2,00 |
| Isohexadecane | 6,00 |
| Dicaprylyl Ether | 4,00 |
| Stearyl Dimethicone | 3,00 |
| Shea Butter | 5,00 |
| Oxynex^{®} 2004 | 0,05 |
| Medialan^{®} LD | 2,00 |
| Phenonip^{®} | 0,90 |
| Aqua purificata | 3,00 |
| Fucogel^{®} 1000 | 3,00 |
| Merquat^{®} Plus 3330 | 1,00 |
| **SUMME** | **100,00** |

### Beispiel 2

| **Rohstoffe** | **100% Rezeptur** |
|---|---|
| Aqua purificata | 59,96 |
| Trilon^{®} BD | 0,05 |
| Glycerin | 7,00 |
| Zitronensäure | 0,04 |
| Abil^{®} Care 85 | 1,00 |
| Cetearyl Alkohol | 2,00 |
| Isohexadecane | 6,00 |
| Dicaprylyl Ether | 4,00 |
| Stearyl Dimethicone | 3,00 |
| Shea Butter | 5,00 |
| Oxynex^{®} 2004 | 0,05 |
| Medialan^{®} LD | 4,00 |
| Phenonip^{®} | 0,90 |
| Aqua purificata | 3,00 |
| Fucogel^{®} 1000 | 3,00 |
| Merquat^{®} Plus 3330 | 1,00 |
| **SUMME** | **100,00** |

### Beispiel 3

| **Rohstoffe** | **100% Rezeptur** |
|---|---|
| Aqua purificata | 58,46 |
| Trilon^{®} BD | 0,05 |
| Glycerin | 7,00 |
| Zitronensäure | 0,04 |
| Abil^{®} Care 85 | 1,00 |
| Cetearyl Alkohol | 2,00 |
| Isohexadecane | 6,00 |
| Dicaprylyl Ether | 4,00 |
| Stearyl Dimethicone | 3,00 |
| Shea Butter | 5,00 |
| Oxynex^{®} 2004 | 0,05 |
| Hostapon^{®} CCG | 2,00 |
| Medialan^{®} LD | 2,00 |
| PNC^{®} 430 | 0,50 |
| Phenonip^{®} | 0,90 |
| Aqua purificata | 3,00 |
| Fucogel^{®} 1000 | 3,00 |
| Merquat^{®} Plus 3330 | 1,00 |
| Dry Flo^{®} AF | 1,00 |
| **SUMME** | **100,00** |

### Beispiel 4

| **Rohstoffe** | **100% Rezeptur** |
|---|---|
| Aqua purificata | 65,71 |
| Trilon^{®} BD | 0,05 |
| Glycerin | 7,00 |
| Zitronensäure | 0,04 |
| Xanthan Gum | 0,50 |
| Abil^{®} Care 85 | 0,50 |
| Cetearyl Alkohol | 2,50 |
| Dicaprylyl Dimethicone | 3,00 |
| Shea Butter | 5,00 |
| Traubenkemöl | 1,00 |
| Tocopherolacetat | 1,00 |
| Oxynex^{®} 2004 | 0,05 |
| Hostapon^{®} CCG | 2,00 |
| Medialan^{®} LD | 2,00 |
| Aristoflex^{®} AVC | 0,25 |
| Phenonip^{®} | 0,90 |
| Aqua purificata | 3,00 |
| Seanamin^{®} SU | 3,00 |
| Merquat^{®} Plus 3330 | 1,00 |
| Dry Flo^{®} AF | 1,00 |
| Parfümöl | 0,50 |
| **SUMME** | **100,00** |

### Beispiele 5

| | |
|---|---|
| **Rohstoffe** | **100% Rezeptur** |
| Aqua purificata | 61,96 |
| Trilon^{®} BD | 0,05 |
| Glycerin | 7,00 |
| Zitronensäure | 0,04 |
| Abil^{®} Care 85 | 1,00 |
| Cetearyl Alkohol | 2,00 |
| Isohexadecane | 6,00 |
| Dicaprylyl Ether | 4,00 |
| Stearyl Dimethicone | 3,00 |
| Shea Butter | 5,00 |
| Oxynex^{®} 2004 | 0,05 |
| Wallasol^{®} L-29 | 2,00 |
| Phenonip^{®} | 0,90 |
| Aqua purificata | 3,00 |
| Fucogel^{®} 1000 | 3,00 |
| Merquat^{®} Plus 3330 | 1,00 |
| **SUMME** | **100,00** |

### Beispiel 6

| **Rohstoffe** | **100% Rezeptur** |
|---|---|
| Aqua purificata | 61,96 |
| Trilon^{®} BD | 0,05 |
| Glycerin | 7,00 |
| Zitronensäure | 0,04 |
| Abil^{®} Care 85 | 1,00 |
| Cetearyl Alkohol | 2,00 |
| Isohexadecane | 6,00 |
| Dicaprylyl Ether | 4,00 |
| Stearyl Dimethicone | 3,00 |
| Shea Butter | 5,00 |
| Oxynex^{®} 2004 | 0,05 |
| Hansanol^{®} NS 243 | 2,00 |
| PHenonip^{®} | 0,90 |
| Aqua purificata | 3,00 |
| Fucogel^{®} 1000 | 3,00 |
| Merquat^{®} Plus 3330 | 1,00 |
| **SUMME** | **100,00** |

### Beispiel 7

| **Rohstoffe** | **100% Rezeptur** |
|---|---|
| Aqua purificata | 70,81 |
| Trilon^{®} BD | 0,05 |
| Glycerin | 7,00 |
| Xanthan Gum | 0,50 |
| Zitronensäure | 0,04 |
| Abil^{®} Care 85 | 1,00 |
| Cetearyl Alkohol | 1,00 |
| Oxynex^{®} 2004 | 0,05 |
| Decyl Oleate | 2,00 |
| Paraffin, dünnflüssig | 8,00 |
| Hostapon^{®} CCG | 1,00 |
| Medialan^{®} LD | 2,00 |
| Phenonip^{®} | 0,90 |
| Aristoflex^{®} AVC | 0,50 |
| Aqua purificata | 3,00 |
| Merquat^{®} Plus 3330 | 1,00 |
| Dry Flo^{®} AF | 1,00 |
| Parfümöl | 0,15 |
| | |
| **SUMME** | **100,00** |

### Beispiel 8

| **Rohstoffe** | **100% Rezeptur** |
|---|---|
| Aqua purificata | 61,96 |
| Trilon^{®} BD | 0,05 |
| Glycerin | 7,00 |
| Zitronensäure | 0,04 |
| Abil^{®} Care 85 | 1,00 |
| Cetearyl Alkohol | 2,00 |
| Isohexadecane | 6,00 |
| Dicaprylyl Ether | 4,00 |
| Stearyl Dimethicone | 3,00 |
| Shea Butter | 5,00 |
| Oxynex^{®} 2004 | 0,05 |
| Hostaphat^{®} KW 340 D | 2,00 |
| Phenonip^{®} | 0,90 |
| Aqua purificata | 3,00 |
| Fucogel^{®} 100 | 3,00 |
| Merquat^{®} Plus 3330 | 1,00 |
| | |
| **SUMME** | **100,00** |

### Beispiel 9

| **Rohstoffe** | **100% Rezeptur** |
|---|---|
| Aqua purificata | 61,96 |
| Trilon^{®} BD | 0,05 |
| Glycerin | 7,00 |
| Zitronensäure | 0,04 |
| Abil^{®} Care 85 | 1,00 |
| Cetearyl Alkohol | 2,00 |
| Isohexadecane | 6,00 |
| Dicaprylyl Ether | 4,00 |
| Stearyl Dimethicone | 3,00 |
| Shea Butter | 5,00 |
| Oxynex^{®} 2004 | 0,05 |
| Brij^{®} 721 | 2,00 |
| Phenonip^{®} | 0,90 |
| Aqua purificata | 3,00 |
| Fucogel^{®} 100 | 3,00 |
| Merquat^{®} Plus 3330 | 1,00 |
| **SUMME** | **100,00** |

### Beispiel 10

| **Rohstoffe** | **100% Rezeptur** |
|---|---|
| Aqua purificata | 79,80 |
| Tween^{®} 80 | 0,20 |
| Abil^{®} Care 85 | 3,00 |
| Caprylic/Capric Triglyceride | 10,00 |
| Isohexadecane | 5,00 |
| Aristoflex^{®} AVC | 0,30 |
| Xanthan Gum | 0,10 |
| Paraffin, dünnflüssig | 1,60 |
| | |
| **SUMME** | **100,00** |

### Beispiel 11

| **Rohstoffe** | **100% Rezeptur** |
|---|---|
| Aqua purificata | 79,80 |
| Tween^{®} 80 | 0,20 |
| | |
| Abil^{®} Care 85 | 3,00 |
| Caprylic/Capric Triglyceride | 10,00 |
| Isohexadecane | 5,00 |
| PNC^{®} 400 | 0,30 |
| Xanthan Gum | 0,10 |
| Paraffin, dünnflüssig | 1,60 |
| **SUMME** | **100,00** |

Die besondere Feinverteilung der erfindungsgemäßen Zusammensetzung wurde anhand der Verschäumbarkeit untersucht.

Die anmeldungsgemäßen Produkte entsprechend den Beispielen 1 bis 7 zeigen wie beschrieben eine gute Schaumbildung mit kleinporigem und nicht schnell zusammenfallendem Schaum sowie Stabilität.

Die Zusammensetzungen gemäß den beigefügten Beispielen 8 bis 11 wurden hergestellt nach dem auf Seite 17 der vorliegenden Beschreibung genannten Verfahren.

Diese Beispiele 8 bis 11 sind Vergleichsprodukte gemäß Stand der Technik. Wie für die Beispiele 1 bis 7 wurde deren Verhalten beim Austragen aus einer Aerosolvorrichtung mit Treibgas überprüft. Dabei wurde (durch visuelle Beobachtung) jeweils ein großporiger flüssiger Schaum erhalten, der schnell zusammenfiel. Die Beispiele 8 und 9 zeigen dabei Produkte mit dem Polysiloxan-Emulgator Abil® Care 85 gemäß Erfindung, aber ohne anionisches bzw. kationisches Tensid, sondern mit dem nichtionischen Tensid Tristeareth-4 Phosphate (Hostaphat® KW 340 D) bzw. Steareth - 21 (Brij® 721) gemäß Stand der Technik wie in der Anmeldung Seite 5, Abs. 2 für Abil® beschrieben. In Beispiel 10 ist als nicht ionisches Tensid Tween 80 und weiterhin als Stabilisator ein Polymer auf Acrylatbasis, nämlich Aristoflex AVC® wie in der EP B 0835 647 (Stand der Technik wie in der Anmeldung auf Seite 3, Abs. 2 beschrieben) enthalten. In Beispiel 11 wurde als nichtionisches Tensid Tween 80 gewählt. Hieraus ist ersichtlich, dass im Gegensatz zum Stand der Technik stabile Schaumzusammensetzungen überraschenderweise nur dann erhalten werden können, wenn bestimmte Polysiloxan- Emulgatoren auf O/W- Basis eingesetzt werden, die dann gerade nicht mit den bisher als zwingend erforderlichen Co-Emulgatoren oder auch bestimmten Stabilisatoren kombiniert werden. Dies war nicht zu erwarten, da der Stand der Technik genau das Gegenteil lehrt, nämlich dass insbesondere derartige Substanzen als zwingend erforderlich für stabile Emulsionen sein sollen, vor allem wenn sie heiß hergestellt werden.

## Patentansprüche

1. Verschäumbare Zusammensetzung auf Basis einer O/W- Emulsion, **dadurch gekennzeichnet, dass** sie 2 bis 50% einer Ölphase, 0,1 bis 10 % eines oder mehrerer O/W- Polysiloxan - Polyether Copolymer- Emulgatoren, ausgewählt aus einem oder mehreren Polyethylenglykol (PEG)-/ Polypropylenglykol(PPG)-Polysiloxan - Copolymeren mit jeweils 1-20 PEG, PPG- Einheiten bezogen auf einen Polyetherblock im Verhältnis 1:0 bis 0,9:1, 0,1 bis 20% eines oder mehrerer anionischer Tenside, 0 bis 40% Zusatzstoffe und als Rest Wasser aufweist.

2. Zusammensetzung gemäß Anspruch 1, **dadurch gekennzeichnet, dass** die Polysiloxan-Polyether Copolymere ausgewählt sind aus PEG/PPG-20/6 Dimethicone, Bis-PEG/PPG-20/20 Dimethicone, PEG-12 oder PEG-14 Dimethicone, PEG/PPG-14/4 oder 14/12 oder 14/14 oder 20/20 oder 18/18 oder 17/18 oder 15/15-Dimethicone oder Bis-PEG/PPG-16/16 PEG/PPG-16/16 Dimethicone oder Mischungen hiervon.

3. Zusammensetzung gemäß einem der Ansprüche 1 oder 2, **dadurch gekennzeichnet, dass** als Tensid ein oder mehrere anionisch(e)s Tensid(e), ausgewählt aus (Alkyl)Sarcosinaten, Alkylsulfaten, Alkylethersulfaten, (Alkyl)Sulfosuccinaten, (Alkyl)Sulfosuccinnamaten, Isethionate oder Mischungen hiervon enthalten sind.

4. Zusammensetzung gemäß Anspruch 3, **dadurch gekennzeichnet, dass** als anionisches Tensid ein oder mehrere Sarcosinate, ein oder mehrere Sulfosuccinate, oder Mischungen hiervon enthalten sind.

5. Zusammensetzung gemäß einem der Ansprüche 1 bis 4, **dadurch gekennzeichnet, dass** der oder die Emulgatoren in einer Gesamtmenge von 0,5 bis 5% und das oder die Tenside in einer Gesamtmenge von 0,5 bis 15% enthalten sind.

6. Zusammensetzung gemäß einem der Ansprüche 1 bis 5, **dadurch gekennzeichnet, dass** 3 bis 35 % Ölphase enthalten ist.

7. Zusammensetzung gemäß einem der Ansprüche 1 bis 6, **dadurch gekennzeichnet, dass** als Ölphase eine oder mehrere Substanzen, ausgewählt aus Kohlenwasserstoffen, Fettalkoholen, Fettalkoholethern, Fettsäureestern, Triglyceriden, natürlichen Fetten und Ölen, Silikonölen sowie Wachsen enthalten sind.

8. Zusammensetzung gemäß einem der Ansprüche 1 bis 7, **dadurch gekennzeichnet, dass** 0,01 bis 25% Zusatzstoffe, ausgewählt aus Wirkstoffen, Stabilisatoren, Konservierungsmitteln, Farbstoffen, Parfümstoffen, Konsistenzreglem, Pflegestoffen, Co- Tensiden, enthalten sind.

9. Zusammensetzung gemäß Anspruch 8, **dadurch gekennzeichnet, dass** die Zusatzstoffe ausgewählt sind aus Fettalkoholen, Acrylatpolymeren, Co-Tensiden , Cellulose- Derivate, Stärke- Derivaten und/oder Feuchthaltemittel, Konditioniermittel.

10. Zusammensetzung gemäß einem der Ansprüche 1 bis 9, **dadurch gekennzeichnet, dass** sie in Form eines Schaumes vorliegt.

11. Verfahren zur Herstellung einer Zusammensetzung gemäß einem der Ansprüche 1 bis 10, **dadurch gekennzeichnet, dass** man die Ölphase, den oder die Emulgatoren, ggf. zusammen mit temperaturstabilen öllöslichen Zusatzstoffen mischt und ggf. auf 50 bis 85° C erhitzt und sodann die wässrige, ggf. auf 50 bis 85° C erwärmte Phase, enthaltend Wasser, das oder die Tenside und ggf. temperaturstabile Zusatzstoffe, unter Rühren hinzufügt, anschließend die entstandene Emulsion ggf. abkühlt und, sofern vorhanden, temperaturlabile Zusatzstoffe hinzufügt.

12. Nicht-therapeutische Verwendung einer Zusammensetzung gemäß einem der Ansprüche 1 bis 10 zur Pflege, zur kosmetischen Behandlung oder milden Reinigung von Haut.

13. Verwendung einer Zusammensetzung gemäß einem der Ansprüche 1 bis 10 zur Herstellung eines Mittels zur dermatologischen Behandlung von dysfunktionaler Haut.

## Claims

1. A foamable composition based on an O/W emulsion, **characterised in that** it comprises 2 to 50% of an oil phase, 0.1 to 10% of one or more O/W polysiloxane-polyether copolymer emulsifiers selected from one or more polyethylene glycol (PEG)-/polypropylene glycol (PPG)-polysiloxane copolymers with in each case 1-20 PEG, PPG units relative to a polyether block in a ratio of 1:0 to 0.9:1, 0.1 to 20% of one or more anionic surfactants, 0 to 40% of additives and water as the remainder.

2. A composition according to claim 1, **characterised in that** the polysiloxane-polyether copolymers are selected from PEG/PPG-20/6 dimethicone, bis-PEG/PPG-20/20 dimethicone, PEG-12 or PEG-14 dimethicone, PEG/PPG-14/4 or 14/12 or 14/14 or 20/20 or 18/18 or 17/18 or 15/15 dimethicone or bis-PEG/PPG-16/16 PEG/PPG-16/16 dimethicone or mixtures thereof.

3. A composition according to either of claim 1 or claim 2, **characterised in that** it contains one or more anionic surfactant(s), selected from (alkyl) sarcosinates, alkyl sulfates, alkyl ether sulfates, (alkyl) sulfosuccinates, (alkyl) sulfosuccinnamates, isethionates or mixtures thereof as the surfactant.

4. A composition according to claim 3, **characterised in that** it contains one or more sarcosinates, one or more sulfosuccinates or mixtures thereof as the anionic surfactant.

5. A composition according to any one of claims 1 to 4, **characterised in that** it contains the emulsifier(s) in a total quantity of 0.5 to 5% and the surfactant(s) in a total quantity of 0.5 to 15%.

6. A composition according to any one of claims 1 to 5, **characterised in that** it contains 3 to 35% of oil phase.

7. A composition according to any one of claims 1 to 6, **characterised in that** it contains one or more substances selected from hydrocarbons, fatty alcohols, fatty alcohol ethers, fatty acid esters, triglycerides, natural fats, and oils, silicone oils and waxes as the oil phase.

8. A composition according to any one of claims 1 to 7, **characterised in that** it contains 0.01 to 25% of additives selected from active ingredients, stabilisers, preservatives, dyes, perfume substances, consistency regulators, conditioners, co-surfactants.

9. A composition according to claim 8, **characterised in that** the additives are selected from fatty alcohols, acrylate polymers, co-surfactants, cellulose derivatives, starch derivatives and/or humectants, conditioners.

10. A composition according to any one of claims 1 to 9, **characterised in that** it assumes the form of a foam.

11. A method for producing a composition according to any one of claims 1 to 10, **characterised in that** the oil phase, the emulsifier(s), optionally together with temperature-stable, oil-soluble additives, are mixed and optionally heated to 50 to 85°C and then the aqueous phase, optionally heated to 50 to 85°C, containing water, the surfactant(s) and optionally temperature-stable additives, is stirred in, then the resultant emulsion is optionally cooled and, if present, temperature-labile additives are added.

12. Non-therapeutic use of a composition according to any one of claims 1 to 10 for the care, cosmetic treatment or gentle cleaning of skin.

13. Use of a composition according to any one of claims 1 to 10 for producing an agent for the dermatological treatment of dysfunctional skin.

## Revendications

1. Composition moussante à base d'une émulsion huile/eau, **caractérisée en ce qu'**elle présente 2 à 50% d'une phase d'huile, 0,1 à 10% d'un ou plusieurs émulsifiants huile/eau à base de copolymères polysiloxane-polyéther, choisis parmi un ou plusieurs copolymères polyéthylèneglycol (PEG)/polypropylèneglycol(PPG)-polysiloxane avec à chaque fois 1 à 20 motifs PEG, PPG dans la proportion de 1:0 à 0,9 :1 par rapport à un bloc polyéther, 0,1 à 20% d'un ou plusieurs tensioactifs anioniques, 0 à 40% d'additifs et en complément de l'eau.

2. Composition selon la revendication 1, **caractérisée en ce que**, les copolymères polysiloxane-polyéther sont choisis parmi les diméthicones PEG/PPG-20/6, les diméthicones bis-PEG/PPG-20/20, les diméthicones PEG-12 ou PEG-14, les diméthicones PEG/PPG-14/4 ou 14/12 ou 14/14 ou 20/20 ou 18/18 ou 17/18 ou 15/15 ou les diméthicones bis-PEG/PPG-16/16 PEG/PPG-16/16 ou leurs mélanges.

3. Composition selon l'une quelconque des revendications 1 ou 2, **caractérisée en ce que**, à titre de tensioactifs, un à plusieurs tensioactifs anioniques choisis parmi les alkylsarcosinates, les alkylsulfates, les alkyléthersulfates, les (alkyl)sulfosuccinates, les (alkyl)sulfosuccinamates, les iséthionates ou leurs mélanges sont contenus.

4. Composition selon la revendication 3, **caractérisée en ce que**, à titre de tensioactifs anioniques, un ou plusieurs sarcosinates, un ou plusieurs sulfosuccinates, ou leurs mélanges sont contenus.

5. Composition selon l'une quelconque des revendications 1 à 4, **caractérisée en ce que** le ou les émulsifiants sont contenus en une quantité totale de 0,5 à 5% et que le ou les tensioactifs sont contenus en une quantité totale de 0,5 à 15%.

6. Composition selon l'une quelconque des revendications 1 à 5, **caractérisée en ce que** 3 à 35% d'une phase d'huile est contenue.

7. Composition selon l'une quelconque des revendications 1 à 6, **caractérisée en ce que**, à titre de phase d'huile, une ou plusieurs substances sont contenues, choisies parmi les hydrocarbures, les alcools gras, les éthers d'alcools gras, les esters d'acides gras, les triglycérides, les graisses et huiles naturelles, les huiles de silicone ainsi que les cires.

8. Composition selon l'une quelconque des revendications 1 à 7, **caractérisée en ce que** 0,01 à 25% d'additifs sont contenus, choisis parmi les substances actives, les stabilisants, les agents conservateurs, les colorants, les substances de parfum, les régulateurs de consistance, les substances de soin, les co-tensioactifs.

9. Composition selon la revendication 8, **caractérisée en ce que** les additifs sont choisis parmi les alcools gras, les polymères acrylate, les co-tensioactifs, les dérivés de cellulose, les dérivés d'amidon et/ou les humectants, les agents de conditionnement.

10. Composition selon l'une quelconque des revendications 1 à 9, **caractérisée en ce qu'**elle existe sous forme d'une mousse.

11. Procédé pour préparer une composition selon l'une quelconque des revendications 1 à 10, **caractérisé en ce que** l'on mélange la phase d'huile, le ou les émulsifiants, éventuellement conjointement avec des additifs liposolubles stables en température, et qu'on les chauffe éventuellement à 50-85°C et que l'on introduit ensuite la phase aqueuse réchauffée à 50-85°C contenant de l'eau, le ou les tensioactifs et éventuellement les additifs stables en température, sous agitation, que l'on refroidit éventuellement ensuite l'émulsion formée et que l'on ajoute des additifs labiles en température dans la mesure où ils sont disponibles.

12. Utilisation non thérapeutique d'une composition selon l'une quelconque des revendications 1 à 10, pour le soin, pour le traitement cosmétique ou nettoyage doux de la peau.

13. Utilisation d'une composition selon l'une quelconque des revendications 1 à 10 pour préparer un agent destiné au traitement dermatologique de la peau dysfonctionnelle.
